# EUROPEAN PATENT SPECIFICATION

(11) **EP 4 259 002 B1**
(45) Date of publication and mention of the grant of the patent: **21.10.2026**
(21) Application number: 21827222.7
(22) Date of filing: 08.12.2021
(51) Int. Cl.: A61B 10/04, A61B 10/02, A61B 18/02

(54) **CARDIAC TISSUE SAMPLING APPARATUS**
VORRICHTUNG ZUR ENTNAHME VON HERZGEWEBEPROBEN
APPAREIL DE PRÉLÈVEMENT DE TISSU CARDIAQUE

(30) Priority: 08.12.2020 US 202063122712 P
(43) Date of publication of application: 18.10.2023
(73) Proprietor: TCEB Limited, Cambridge CB4 0WS (GB)
(72) Inventor: SHARP, Alexander, Cambridge CB2 9BW (GB); GRACE, Andrew, Cambridge CB3 0EJ (GB)
(74) Representative: Potter Clarkson
(86) International application number: PCT/IB2021/061459
(87) International publication number: WO 2022/123458

(56) References cited:
- WO-A1-2019/168634
- WO-A2-2008/023193
- US-A1- 2013 165 915
- US-A1- 2016 066 896

## Description

### CROSS-REFERENCE TO RELATED APPLICATION

### FIELD OF INTEREST

The present inventive concepts relate to the field of tissue sampling in living beings, and is particularly useful for tissue sampling in living mammals.

### BACKGROUND

In various medical diagnostic, treatment, and research contexts it is particularly useful to be able to sample tissue for testing and analysis. As examples, clinical uses in the field of cardiology of tissue sampling include, but are not limited to, testing tissue of transplant recipients to assess for possible rejection, and diagnosing various maladies, e.g., cardiac sarcoid, amyloidosis, subtypes of myocarditis, intracardiac tumors. As further examples, research uses of tissue sampling include, but are not limited to, transcriptomics - multiple and single cell and Das 2019 - Cardiac biopsies in CABG patients - Transcriptomics of HFpEF.

In 1972, Caves modified the Konno biopsy for use through the right internal jugular vein. This modification allowed the bioptome to be inserted percutaneously, but the large diameter of the bioptome head required use of a large (non-valved) sheath that placed the patient at risk for bleeding or air embolization at the time of bioptome insertion or removal. The technique did allow several advantages, including percutaneous insertion, use of local anesthetic allowing minimal discomfort to the patient, rapid performance, direct passage of the bioptome to the right ventricular apex, and repeated entry and exit through the same sheath.

Caves subsequently introduced the Stanford modification to the previous Konno bioptome. The Stanford (or Caves-Shulz) bioptome included two hemispheric cutting jaws with a combined diameter of 3 mm (9F) mounted on the catheter tip. One of the jaws remained stationary while the other opened and closed under the control of a mosquito-like clamp at the proximal end of the catheter used to extract tissue. Spring-loaded adjustable nuts allowed the operator to adjust the amount of force applied with opening and closing of the surgical-like clamp. Because this bioptome was reusable, it required careful cleaning after each use and ultimately needed retooling and sharpening of the cutting edges of the jaws after 50 procedures.

However, significant limitations are associated with the foregoing apparatus and approach. For example, using a bioptome catheter had a 0.54% risk of major complications, e.g., perforation. This risk increased when not sampling interventricular septum. Additionally, the jaws have a tendency to crush the sampled tissue, which can be negatively impactful in transbronchial lung cryobiopsy which has been used to sample lung tissue in the diagnosis of lung disease. The technique is performed by passing a probe through the working channel of a bronchoscope out to the lung periphery. The probe tip is rapidly cooled until cryoadhesion occurs between the tip and adjacent lung tissue. A tissue sample is extracted by firmly pulling on the probe tip. The probe is then removed with bronchoscope from the airway.

Cryobiopsy has not been used to sample heart tissue. Sampling heart tissue within a cardiac chamber, which cannot be simply performed through an airway, poses challenges not encountered with lung cryobiopsy. Being able to remove only tissue cells from the cardiac wall would be particularly beneficial, doing minimal damage, rather than adhering and removing chunks of tissue pulled from a lung wall.
US 2013/165915 describes comprehensive systems, devices and methods for delivering energy to tissue for a wide variety of applications, including medical procedures (e.g., tissue ablation, resection, cautery, vascular thrombosis, treatment of cardiac arrhythmias and dysrhythmias, electrosurgery, tissue harvest, etc.).
US 2016/066896 describes cryogenic biopsy assemblies that comprise a tissue acquisition device and cryogenic probe. The cryogenic biopsy assembly may be inserted into a lumen of an endoscope, the endoscope may be manipulated to position the cryogenic biopsy assembly at the desired tissue acquisition site. Flow of refrigerant to and from the cryogenic biopsy assembly may be controlled from a control unit. Also disclosed are systems containing such cryogenic biopsy assemblies and methods cryogenic biopsy that employ such cryogenic biopsy assemblies.
WO 2019/168634 describes a device for cryogenic biopsy sampling, the device including: a dual lumen tube; an elongated probe tip coupled to the distal end of the dual lumen tube, with a first lumen extending into a hollow portion in the tip and a second lumen in fluid communication with the hollow portion; a first port in fluid communication with the first lumen; a second port in fluid communication with the second lumen, wherein the first lumen, the elongated probe element, and the second lumen provide a closed pathway through which a substance introduced through the first port can flow through the first lumen into the elongated probe element, and out of the elongated probe element through the second lumen to the second port.
WO 2008/023193 A2 discloses a device for cryogenic biopsy sampling. The device including: a dual lumen tube; an elongated probe tip coupled to the distal end of the dual lumen tube, with a first lumen extending into a hollow portion in the tip and a second lumen in fluid communication with the hollow portion; a first port in fluid communication with the first lumen; a second port in fluid communication with the second lumen, wherein the first lumen, the elongated probe element, and the second lumen provide a closed pathway through which a substance introduced through the first port can flow through the first lumen into the elongated probe element, and out of the elongated probe element through the second lumen to the second port.

### SUMMARY

Aspects of the invention are as set out in the appended claims.

In accordance with an aspect of the inventive concept, provided is a cardiac cryobiopsy apparatus or system. The apparatus comprises: a cryoprobe having a cryogenic probe tip and configured for introduction into a cardiac chamber via a blood vessel; the cryoprobe having at least one microtube configured to inject a liquid refrigerant into a cavity of the probe tip; a steering mechanism configured to steer the probe tip to a tissue harvesting site within the cardiac chamber; and a controller comprising a processor. The controller is configured to bring the probe tip to a temperature at or below freezing to adhere cardiac tissue and/or cells to the probe tip by injecting the liquid refrigerant into the probe tip and maintain the probe tip a temperature at or below freezing by injecting the liquid refrigerant into the probe tip through retraction of the probe tip from the body to harvest the adhered cardiac tissue and/or cells.

In some embodiments, the controller is further configured to raise the temperature of the probe tip to above freezing to release the harvested cardiac tissue or cells from the probe tip once the probe tip is removed from the body.

In some embodiments, the probe tip, or a portion thereon, is removable with the harvested cardiac tissue or cells from the probe once the probe tip is removed from the body.

In some embodiments, the cryoprobe further includes a proximal end comprising an inlet port and an outlet port and at least one microtube configured to inject a liquid refrigerant received via the inlet port into the probe tip, wherein the probe tip defines a chamber configured to receive the liquid refrigerant and transition the liquid refrigerant from a liquid state to a gas state to lower the probe tip temperature, and wherein the probe defines a gas evacuation path from the probe tip to the outlet port.

In some embodiments, the controller is further configured to control the temperature of the probe tip.

In some embodiments, the probe tip includes at least one sensor coupled to the processor and configured to sense at least one condition at the probe tip.

In some embodiments, the at least one condition at the probe tip is a temperature and/or presence of a tissue sample.

In some embodiments, the controller and the at least one sensor are configured to sense a temperature of the probe tip and/or refrigerant entering and/or exiting the probe tip.

In some embodiments, the controller and the at least one sensor are configured to sense the presence and/or absence of the cardiac tissue or cells adhered to the probe tip.

In some embodiments, the controller and the at least one sensor are configured to sense the presence and/or absence of the cardiac tissue or cells adhered to the probe tip using an optical sensor.

In some embodiments, the apparatus further comprises a feedback loop between the at least one sensor and the controller to enable the controller to monitor and regulate the probe tip temperature.

In some embodiments, the controller is configured to maintain the probe tip temperature at or below freezing after exiting the body for at least 10 seconds, at least 30 seconds, or at least 1 minute.

In some embodiments, the controller is configured to maintain the probe tip temperature at or below freezing after exiting the body until a user input received via a user interface directs the controller to enable to probe tip temperature to rise above freezing.

In some embodiments, the controller comprises an ambient temperature sensor and the controller is further configured to set the probe tip temperature within the cardiac chamber based on a sensed ambient temperature outside the body.

In some embodiments, the controller is further configured to set the probe tip temperature below freezing based on the sensed ambient temperature and a duration of time the probe tip is to be at or below freezing outside the body.

### BRIEF DESCRIPTION OF THE DRAWINGS

The present inventive concepts will become more apparent in view of the attached drawings and accompanying detailed description. The embodiments depicted therein are provided by way of example, not by way of limitation, wherein like reference numerals refer to the same or similar elements. The drawings are not necessarily to scale, emphasis instead being placed upon illustrating aspects of the inventive concepts.

In the drawings:
FIG. 1 is a representation of endoscopic cryoprobes in the prior art;
FIG. 2 is a representation of a cryoablation probe in the prior art;
FIG. 3 is a diagram illustrating a cryobiopsy tissue sampling apparatus, in accordance with aspects of the inventive concepts;
FIG. 3A is an example embodiment of a probe tip with tissue sensor, in accordance with aspects of the inventive concepts; and
FIG. 4 is a is a flowchart of a cardiac cryobiopsy method, in accordance with aspects of the inventive concepts.

### DETAILED DESCRIPTION OF PREFERRED EMBODIMENTS

Various aspects of the inventive concepts will be described more fully hereinafter with reference to the accompanying drawings, in which some exemplary embodiments are shown. The present inventive concept may, however, be embodied in many different forms and should not be construed as limited to the exemplary embodiments set forth herein.

It will be understood that, although the terms first, second, etc. are be used herein to describe various elements, these elements should not be limited by these terms. These terms are used to distinguish one element from another, but not to imply a required sequence of elements. For example, a first element can be termed a second element, and, similarly, a second element can be termed a first element, without departing from the scope of the present invention. As used herein, the term "and/or" includes any and all combinations of one or more of the associated listed items.

It will be understood that when an element is referred to as being "on" or "connected" or "coupled" to another element, it can be directly on or connected or coupled to the other element or intervening elements can be present. In contrast, when an element is referred to as being "directly on" or "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, steps, operations, elements, and/or components, but do not preclude the presence or addition of one or more other features, steps, operations, elements, components, and/or groups thereof.

Spatially relative terms, such as "beneath," "below," "lower," "above," "upper" and the like may be used to describe an element and/or feature's relationship to another element(s) and/or feature(s) as, for example, illustrated in the figures. It will be understood that the spatially relative terms are intended to encompass different orientations of the device in use and/or operation in addition to the orientation depicted in the figures. For example, if the device in the figures is turned over, elements described as "below" and/or "beneath" other elements or features would then be oriented "above" the other elements or features. The device may be otherwise oriented (e.g., rotated 90 degrees or at other orientations) and the spatially relative descriptors used herein interpreted accordingly.

Exemplary embodiments are described herein with reference to cross-sectional illustrations that are schematic illustrations of idealized exemplary embodiments (and intermediate structures). As such, variations from the shapes of the illustrations as a result, for example, of manufacturing techniques and/or tolerances, are to be expected. Thus, exemplary embodiments should not be construed as limited to the particular shapes illustrated herein, but are to include deviations in shapes that result, for example, from manufacturing or reasonably foreseeable alternatives.

To the extent that functional features, operations, and/or steps are described herein, or otherwise understood to be included within various embodiments of the inventive concept, such functional features, operations, and/or steps can be embodied in functional blocks, units, modules, operations and/or methods. And to the extent that such functional blocks, units, modules, operations and/or methods include computer program code, such computer program code can be stored in a computer readable medium, e.g., such as non-transitory memory and media, that is executable by at least one computer processor.

In accordance with the invention, provided is a cardiac cryobiopsy (or cryogenic boipsy) apparatus.

The cardiac cryobiopsy apparatus can be used to remove a cluster of tissue cells, which can be a few tissue cells, rather than a relatively crude chunk of cardiac tissue, thereby causing minimal damage and disruption to the cardiac tissue at the harvesting site. Therefore, the sampled tissue (or harvested tissue) can include a small number of tissue cells from the harvest site.

In various embodiments, a cryobiopsy apparatus can include an intravenous probe having at least one probe tip capable of reaching temperatures sufficient to freeze tissue such that the tissue or cells adhere to the tip. The tip remains at the freezing temperature through retraction of the probe so that the adhered tissue sample can be maintained on the probe tip through the complete retraction or removal process, from harvest site to at least the point of exiting the body or beyond, so that the harvested sample can be collected for its intended purpose. In some embodiments, the temperature of the probe tip is selectively maintained at below freezing even after the point of exiting the body, e.g., until a clinician raises the probe tip temperature above freezing to release the tissue sample from the probe tip.

In some embodiments, the probe tip temperature can be brought sufficiently low so that the temperature of the probe tip does not immediately rise above freezing in ambient room temperature. For example, in some embodiments, the probe tip temperature upon exit from the body can be sufficiently below freezing that the probe tip does not rise above the freezing temperature after exit for a freeze time *(t).* In some embodiments, *t* is at least one (1) minute, *t is* at least thirty (30) seconds, and/or t is at least ten (10) seconds. In some embodiments, a controller coupled to the probe can sense the ambient temperature, using an ambient temperature sensor 20, external to the body and adjust the probe tip temperature to achieve a desired freeze time t once the probe tip is removed from the body.

Cryoablation probes have been used to ablate tissue within a heart to remediate an arrhythmia by killing heart tissue through freezing (cell death). Some factors affecting cell death include: freezing temperature, freezing rate, freezing duration, thaw rate, and repeat freezing. The mechanism of cell death includes direct cellular damage, vascular failure, and/or immunological effect.

In such cryoablation systems, a probe tip is brought into contact with the endocardium and brought to a controlled freezing temperature intended to freeze a portion of the inner wall of the heart to a prescribed depth needed to kill the desired tissue to remedy the arrhythmia. Once the treatment is complete, and cell death has occurred, the probe tip is returned to a temperature above freezing and the probe is removed through the catheter sheath used for insertion. Cardiac wall tissue is not collected from the probe tip.

Unlike cryoablation, a cryobiopsy is fundamentally intended to avoid cell death and it is not a treatment therapy. Cryobiopsy, in accordance with the present inventive concepts, seeks to remove a sample of living tissue cells for subsequent analysis and testing. Because of the diametrically opposed goals of cryoablation and cryobiopsy, even if somewhat similar devices can be used in each, the method of using them would be dramatically different. In addition, setup and configuration of the cryo device could be different when used for different purposes. For example, the cryo device (or system) may have a controller implementing a different set of logic and computer program instructions to achieve a cryobiopsy rather than a cryoablation. In some embodiments, there may be different physical attributes and characteristics of a cryobiopsy device (or system) when compared to a cryoablation system, such as different size probe tips and/or different materials used in the probe tips.

In some embodiments, however, the same cryo device could be used for a cryoablation procedure and, then, a cryobiopsy procedure, if implementing appropriate logic for cryobiopsy. For example, the cryo probe can be advanced into the heart and used to perform a cryoablation and, then, the probe can be transitioned for use in performing a cryobiopsy at the ablation site or another site within the heart, but the cryoablation and the cryobiopsy remain to different procedures.

In various embodiments, a cryobiopsy is performed by the moist cardiac tissue at the harvest site freezing to the probe tip. In various embodiments, there may be a formation of ice at the tissue contact side which adheres the catheter probe tip to the tissue. The catheter, with the sampled tissue, is retracted whilst the tissue sample is still attached to the probe tip, which remains at or below freezing temperature through the retraction.

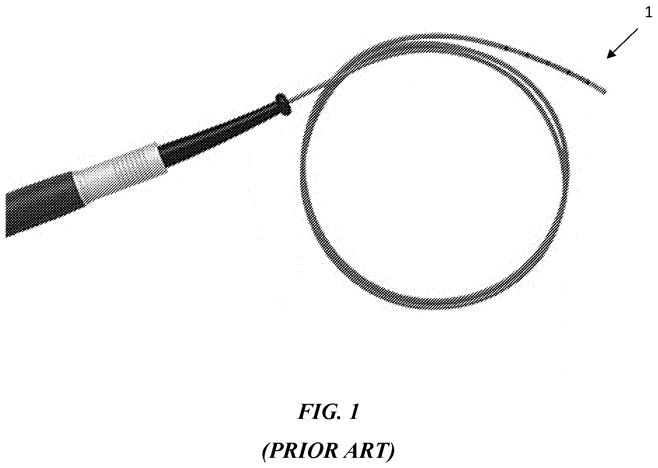

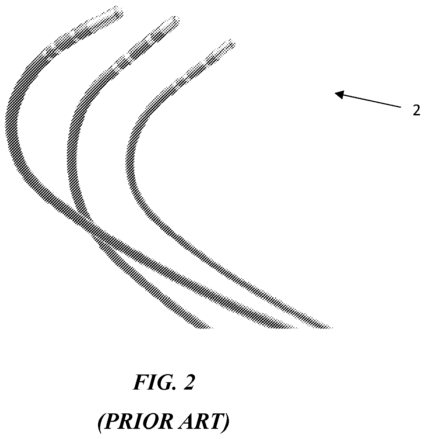

FIG. 1 is a representation of endoscopic cryoprobes in the prior art. FIG. 2 is a representation of a cryoablation probe in the prior art.

Examples of cryoprobes that can be used for a cryobiopsy include, but are not limited to various Flexible Cryoprobes 1 offered by Erbe, such as the 1.1mm, 1.7mm, 1.9mm, 2.4mm diameter, as illustrated in FIG. 1 used for endoscopy and the Freezor^{™} 2 cardiac catheters 4/6/8mm offered by Medtronic for cryoablation, as illustrated in FIG. 2.

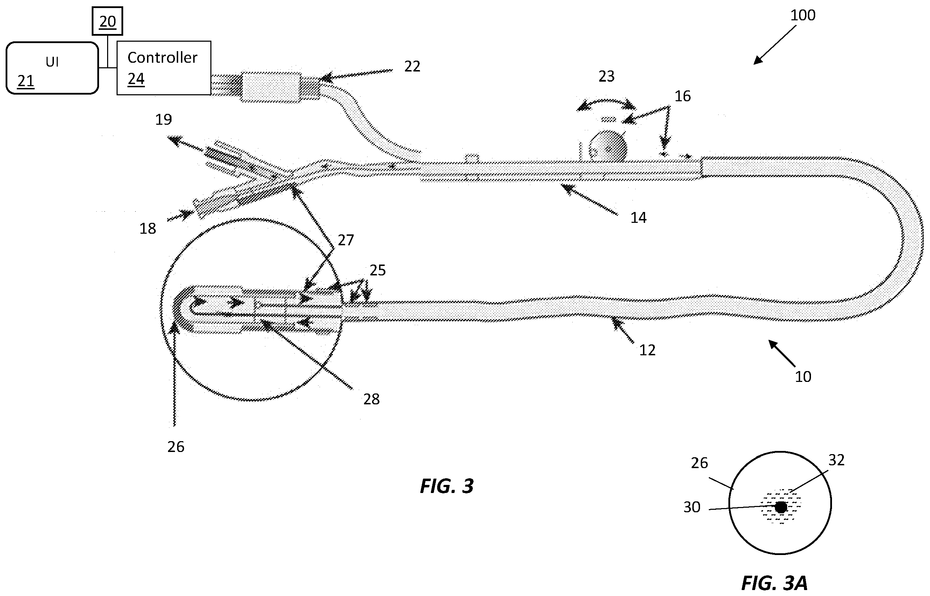

FIG. 3 is a diagram illustrating a cryobiopsy tissue sampling apparatus 100, in accordance with aspects of the inventive concepts. In this embodiment, the apparatus 100 includes an outer sheath for introducing a cryoprobe 10 into the body and, ultimately, into an inner chamber of the heart. The cryoprobe 10 includes an elongate catheter having a proximal end and a distal. The distal end comprises a probe tip 26, and the proximal end comprises a refrigerant inlet port 18 and a refrigerant outlet port 19. Liquid refrigerant 27 is introduced into the probe 10 via the inlet port 18. The liquid refrigerant is injected into the probe tip 26 via at least one micro-tube within the probe 10, wherein the probe tip defines a cavity within which the liquid refrigerant evaporates to transitions the refrigerant from a liquid state to a gas state, thereby lowering the temperature of the probe tip 26. The refrigerant, as gas, is evacuated via a path within the probe 10 from the probe tip to the outlet port 19. The outlet port can be a vacuum port configured to connect to a vacuum that aides the evacuation of the refrigerant gas from the probe tip. The pressure of liquid refrigerant drops as it leaves the microtubes and enters the probe tip. The decrease in pressure in the probe tip causes the state change to from a liquid to a gas.

In some embodiments, the cryoprobe 10 comprises a Flexible 9 French probe 12. A handle 14 includes lever controls 16 that enable steering of the probe 10 inside the body. The lever controls 16 also enable a user to control and manipulate tip curvature, as seen by arrows 23. The probe 10 includes the liquid refrigerant input port 18 and the refrigerant output port 19. The probe 10 can include an electrical connector 22 for driving ring electrodes 25 at a distal probe end. The electrical connector 22 could be coupled to a properly configured controller 24 that includes cryobiopsy logic for controlling a magnitude and a duration of freezing temperatures at the probe tip 26, and for tissue harvesting through extraction external to the probe sheath. The apparatus can include a user interface (UI) 21 that enables a user to interact with the apparatus and the probe 10 via the controller 24.

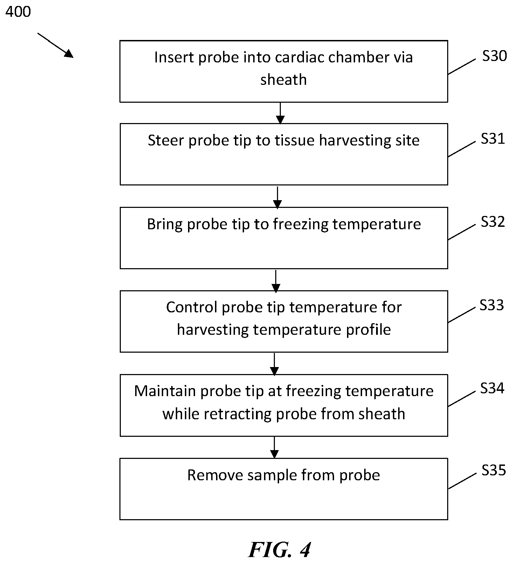

FIG. 4 is a is a flowchart of an embodiment of a cardiac cryobiopsy method 300, however, such a method has not been claimed. The method 400 of FIG. 4 may be implemented with the apparatus of FIG. 3, or the like. According to some embodiments, the cryoprobe 10 is introduced into the body and eventually a chamber of the heart via a sheath (S30). The probe tip 26 is steered to a tissue harvesting site (S31). The probe tip 26 can be brought to the freezing temperature once in contact with the tissue, or just before (S33).

The temperature of the probe tip 26 can be controlled according to a harvesting temperature profile that dictates a temperature magnitude and duration needed to harvest the tissue (or cells) at the site. A profile can have a constant probe tip temperature during harvesting or the temperature can be varied or have different levels during different parts of the process. In some embodiments, the temperature can be controlled by an operator or by a preprogrammed controller. Temperature, surface area, probe tip pressure against the cardiac wall and duration can be parameters used in defining a harvesting profile, and can be monitored and/or controlled at the probe tip. Unlike cryoablation, however, the objective is to harvest live cells, not compromise or damage the tissue at the location to effect ablation.

The probe tip 26 is maintained at the freezing, harvesting temperature during harvesting, but also is maintained at a freezing, retraction temperature after harvesting is complete at the harvesting site, so that the cryoprobe can be retracted via the sheath without releasing the harvested tissue sample due to thawing (S34). The harvesting temperature and the retraction temperature can be the same or different, but are preferably below freezing in various embodiments.

In some embodiments, the probe tip 26 can include at least one sensor 30, e.g., an optical sensor, that indicates if tissue cells were harvested, as shown in FIG. 3A. The sensor 30 can be part of a feedback loop with a controller 24 that influences and/or controls the temperature of the probe tip 26. For example, when the sensor indicates harvesting is complete, an indication can be sent to an operator and/or the controller 24. The probe temperature can then be transitioned to the retraction temperature, if different from the harvesting temperature, by the operator and/or the controller 24, and the probe 10 can be retracted. In various embodiments, if harvested tissue 32 on the probe tip 26 blocks the optical sensor 30, e.g., light is reflected within the probe tip 26, then a tissue sample is determined to be adhered to the probe tip for harvesting.

There can be various approaches to removing the tissue sample frozen to the probe tip 26, once retracted from the body. In various embodiments, once the probe 10 is out of the sheath and body, the probe tip 26 temperature can be transitioned above freezing to release the harvested tissue sample from the probe. In various embodiments, the release of the harvested tissue sample from the probe can include removing the tissue sample or cells from the tissue sample from the probe tip 26. In other embodiments, the release the harvested tissue sample from the probe can include removing the probe tip 26, or a portion thereof, with the tissue sample still attached from the probe 10, such that the tissue sample 32 with probe tip 26, or portion thereof, could be sent with the sample still frozen for analysis, rather than thawing beforehand.

In various embodiments, a non-claimed cardiac cryobiopsy method comprises introducing a cryoprobe into a cardiac chamber of a body (S30); steering a probe tip 26 to a tissue harvesting site (S31); bringing the probe tip 26 to a harvesting temperature below freezing (S33) and maintaining contact until cardiac tissue or cells 32 adheres to the probe tip through freezing (S34); and while maintaining the probe tip at or below freezing, retracting the probe tip from the body (S35).

In some embodiments, the method can further comprise, once the probe tip is removed from the body, raising the temperature of the probe tip to above freezing to release the harvested cardiac tissue or cells.

In some embodiments, the method can further comprise, once the probe tip is removed from the body, removing the probe tip, or a portion thereon, with cardiac tissue or cells adhered thereto from the probe.

In some embodiments, the cryoprobe 10 includes an elongate catheter having a proximal end and a distal end. The distal end can comprise the probe tip 26 and the proximal end can comprise a refrigerant inlet port 18 and a refrigerant outlet port 19. The method can include introducing refrigerant to the probe tip 26 via the inlet port 18 and through the catheter, thereby lowering the temperature of the probe tip, and evacuating the refrigerant via the outlet port 19 and through the catheter shaft. The refrigerant may be output from the outlet port 19 as refrigerant gas.

In some embodiments, the probe tip 26 includes a micro-tube 28 and the method 300 includes injecting the refrigerant as liquid refrigerant into the probe tip 26 via the micro-tube 28, evaporating the refrigerant in the probe tip 26 to lower the probe tip temperature, and evacuating the refrigerant as refrigerant gas from the probe tip 26 and out outlet port 19.

In some embodiments, the method can further comprise controlling the temperature of the probe tip 26 via a controller 24 comprising a processor.

In some embodiments, the probe tip 26 includes at least one sensor coupled to the processor and the method further comprises sensing a temperature of the probe tip 26 and/or refrigerant entering and/or exiting the probe tip 26.

In some embodiments, method further comprises sensing the presence and/or absence of the cardiac tissue or cells adhered to the probe tip 26.

In some embodiments, the method further comprises establishing a feedback loop between the at least one sensor and the controller 24 to enable the controller 24 to monitor and regulate the probe tip 26 temperature.

It is appreciated that certain features of the invention, which are, for clarity, described in the context of separate embodiments, may also be provide in combination in a single embodiment. Conversely, various features of the invention which are, for brevity, described in the context of a single embodiment may also be provided separately or in any suitable subcombination.

The scope of the invention is defined by the appended claims solely.

## Claims

1. A cardiac cryobiopsy apparatus (100), comprising:
a cryoprobe (10) having a cryogenic probe tip (26) configured for introduction into a cardiac chamber via a blood vessel, the cryoprobe (10) having at least one microtube configured to inject a liquid refrigerant (27) into a cavity of the probe tip;
a steering mechanism (16) configured to steer the probe tip (26) to a tissue harvesting site within the cardiac chamber; and
a controller (24) comprising a processor configured to:
bring the probe tip (26) to a temperature at or below freezing to adhere cardiac tissue and/or cells (32) to the probe tip by injecting the liquid refrigerant into the probe tip (26); and
maintain the probe tip (26) temperature at or below freezing by injecting the liquid refrigerant into the probe tip (26) through retraction of the probe tip (26) from the body to harvest the adhered cardiac tissue and/or cells.

2. The apparatus of claim 1, wherein the controller (24) is further configured to:
raise the temperature of the probe tip to above freezing to release the harvested cardiac tissue or cells from the probe tip once the probe tip is removed from the body.

3. The apparatus of claim 1, wherein the probe tip (26), or a portion thereon, is removable with the harvested cardiac tissue or cells from the probe (10) once the probe tip (26) is removed from the body.

4. The apparatus of claim 1, wherein the cryoprobe further includes:
a proximal end comprising an inlet port (18) and an outlet port (19);
the at least one microtube configured to inject the liquid refrigerant received via the inlet port (18) into the probe tip (26),
wherein the probe tip (26) defines a chamber configured to receive the liquid refrigerant from the microtube and the liquid refrigerant transitions from a liquid state to a gas state when the liquid refrigerant leaves the microtube and enters the chamber to lower the probe tip (26) temperature, and
wherein the probe (10) defines a gas evacuation path from the probe tip to the outlet port.

5. The apparatus of claim 1, wherein the controller (24) is further configured to control the temperature of the probe tip (26).

6. The apparatus of claim 5, wherein the probe tip (26) includes at least one sensor (30) coupled to the processor and configured to sense at least one condition at the probe tip (26).

7. The apparatus of claim 6, wherein the at least one condition at the probe tip (26) is a temperature and/or presence of a tissue sample.

8. The apparatus of claim 6, wherein the controller (24) and the at least one sensor (30) are configured to sense a temperature of the probe tip (26) and/or refrigerant entering and/or exiting the probe tip (26).

9. The apparatus of claim 6, wherein the controller (24) and the at least one sensor (30) are configured to sense the presence and/or absence of the cardiac tissue or cells adhered to the probe tip (26).

10. The apparatus of claim 6, wherein the controller (24) and the at least one sensor (30) are configured to sense the presence and/or absence of the cardiac tissue or cells adhered to the probe tip (26) using an optical sensor.

11. The apparatus of claim 6, further comprising a feedback loop between the at least one sensor (30) and the controller (24) to enable the controller (24) to monitor and regulate the probe tip (26) temperature.

12. The apparatus of claim 6, wherein the controller (24) is configured to maintain the probe tip (26) temperature at or below freezing after exiting the body for at least 10 seconds, at least 30 seconds, or at least 1 minute.

13. The apparatus of claim 6, wherein the controller (24) is configured to maintain the probe tip (26) temperature at or below freezing after exiting the body until a user input received via a user interface directs the controller (24) to enable the probe tip (26) temperature to rise above freezing.

14. The apparatus of claim 6, wherein the controller (24) comprises an ambient temperature sensor and the controller (24) is further configured to set the probe tip (26) temperature within the cardiac chamber based on a sensed ambient temperature outside the body.

15. The apparatus of claim 14, wherein the controller (24) is further configured to set the probe tip (26) temperature below freezing based on the sensed ambient temperature and a duration of time the probe tip (26) is to be at or below freezing outside the body.

## Patentansprüche

1. Einrichtung (100) zur Kryobiopsie am Herzen, umfassend:
eine Kryosonde (10) mit einer kryogenen Sondenspitze (26), die zur Einführung in eine Herzkammer über ein Blutgefäß konfiguriert ist, wobei die Kryosonde (10) mindestens ein Mikroröhrchen aufweist, das konfiguriert ist, um ein flüssiges Kältemittel (27) in einen Hohlraum der Sondenspitze einzuspritzen;
einen Lenkmechanismus (16), der konfiguriert ist, um die Sondenspitze (26) zu einer Gewebeentnahmestelle im Inneren der Herzkammer zu lenken; und
eine Steuerung (24), umfassend einen Prozessor, der konfiguriert ist,
um die Sondenspitze (26) auf eine Temperatur auf oder unter dem Gefrierpunkt zu bringen, um Herzgewebe und/oder -zellen (32) an der Sondenspitze anhaften zu lassen, und zwar durch Einspritzen des flüssigen Kältemittels in die Sondenspitze (26); und
um die Temperatur der Sondenspitze (26) durch Einspritzen des flüssigen Kältemittels in die Sondenspitze (26) auf oder unter dem Gefrierpunkt zu halten, und zwar während des Zurückziehens der Sondenspitze (26) aus dem Körper, um das anhaftende Herzgewebe und/oder die anhaftenden Herzzellen zu entnehmen.

2. Einrichtung nach Anspruch 1, wobei die Steuerung (24) ferner konfiguriert ist,
um die Temperatur der Sondenspitze auf über den Gefrierpunkt zu erhöhen, um das entnommene Herzgewebe oder die entnommenen Herzzellen von der Sondenspitze zu lösen, sobald die Sondenspitze aus dem Körper entfernt worden ist.

3. Einrichtung nach Anspruch 1, wobei die Sondenspitze (26) oder ein Teil davon mit dem entnommenen Herzgewebe oder den entnommenen Herzzellen von der Sonde (10) entfernbar ist, sobald die Sondenspitze (26) aus dem Körper entfernt worden ist.

4. Einrichtung nach Anspruch 1, wobei die Kryosonde ferner einschließt:
ein proximales Ende, umfassend eine Einlassöffnung (18) und eine Auslassöffnung (19);
das mindestens eine Mikroröhrchen, das konfiguriert ist, um das über die Einlassöffnung (18) zugeführte flüssige Kältemittel in die Sondenspitze (26) einzuspritzen,
wobei die Sondenspitze (26) eine Kammer umgrenzt, die konfiguriert ist, um das flüssige Kältemittel aus dem Mikroröhrchen aufzunehmen, und das flüssige Kältemittel aus dem flüssigen Zustand in den gasförmigen Zustand übergeht, wenn es das Mikroröhrchen verlässt und in die Kammer eintritt, sodass die Temperatur der Sondenspitze (26) abgesenkt wird, und wobei die Sonde (10) einen Gasabführungsweg von der Sondenspitze zur
Auslassöffnung umgrenzt.

5. Einrichtung nach Anspruch 1, wobei die Steuerung (24) ferner konfiguriert ist, um die Temperatur der Sondenspitze (26) zu steuern.

6. Einrichtung nach Anspruch 5, wobei die Sondenspitze (26) mindestens einen Sensor (30) einschließt, der mit dem Prozessor gekoppelt und konfiguriert ist, um mindestens einen Zustand an der Sondenspitze (26) zu erfassen.

7. Einrichtung nach Anspruch 6, wobei der mindestens eine Zustand an der Sondenspitze (26) eine Temperatur und/oder das Vorhandensein einer Gewebeprobe ist.

8. Einrichtung nach Anspruch 6, wobei die Steuerung (24) und der mindestens eine Sensor (30) konfiguriert sind, um eine Temperatur der Sondenspitze (26) und/oder des Kältemittels, das in die Sondenspitze (26) eintritt und/oder aus dieser austritt, zu erfassen.

9. Einrichtung nach Anspruch 6, wobei die Steuerung (24) und der mindestens eine Sensor (30) konfiguriert sind, um das Vorhandensein und/oder Nichtvorhandensein von an der Sondenspitze (26) anhaftendem Herzgewebe oder anhaftenden Herzzellen zu erfassen.

10. Einrichtung nach Anspruch 6, wobei die Steuerung (24) und der mindestens eine Sensor (30) konfiguriert sind, um das Vorhandensein und/oder Nichtvorhandensein von an der Sondenspitze (26) anhaftendem Herzgewebe oder anhaftenden Herzzellen unter Verwendung eines optischen Sensors zu erfassen.

11. Einrichtung nach Anspruch 6, ferner eine Rückkopplungsschleife zwischen dem mindestens einen Sensor (30) und der Steuerung (24) umfassend, um der Steuerung (24) zu ermöglichen, die Temperatur der Sondenspitze (26) zu überwachen und zu regeln.

12. Einrichtung nach Anspruch 6, wobei die Steuerung (24) konfiguriert ist, um die Temperatur der Sondenspitze (26) nach dem Austreten aus dem Körper für mindestens 10 Sekunden, mindestens 30 Sekunden oder mindestens 1 Minute auf oder unter dem Gefrierpunkt zu halten.

13. Einrichtung nach Anspruch 6, wobei die Steuerung (24) konfiguriert ist, um die Temperatur der Sondenspitze (26) nach dem Austreten aus dem Körper auf oder unter dem Gefrierpunkt zu halten, bis eine über eine Benutzerschnittstelle empfangene Benutzereingabe die Steuerung (24) anweist, zuzulassen, dass die Temperatur der Sondenspitze (26) über den Gefrierpunkt steigt.

14. Einrichtung nach Anspruch 6, wobei die Steuerung (24) einen Umgebungstemperatursensor umfasst und die Steuerung (24) ferner konfiguriert ist, um die Temperatur der Sondenspitze (26) im Inneren der Herzkammer basierend auf einer erfassten Umgebungstemperatur außerhalb des Körpers einzustellen.

15. Einrichtung nach Anspruch 14, wobei die Steuerung (24) ferner konfiguriert ist, um die Temperatur der Sondenspitze (26) basierend auf der erfassten Umgebungstemperatur und einer Zeitdauer, die die Sondenspitze (26) außerhalb des Körpers auf oder unter dem Gefrierpunkt sein soll, unter den Gefrierpunkt einzustellen.

## Revendications

1. Appareil de cryobiopsie cardiaque (100) comprenant :
une cryosonde (10) présentant une pointe de sonde cryogénique (26) conçue pour être introduite dans une cavité cardiaque par le biais d'un vaisseau sanguin, la cryosonde (10) présentant au moins un microtube conçu pour injecter un fluide frigorigène liquide (27) dans une cavité de la pointe de sonde ;
un mécanisme de direction (16) conçu pour diriger la pointe de sonde (26) vers un site de prélèvement de tissu à l'intérieur de la cavité cardiaque ; et
un dispositif de commande (24) comprenant un processeur configuré pour :
amener la pointe de sonde (26) à une température égale ou inférieure à la température de congélation pour faire adhérer le tissu et/ou les cellules cardiaques (32) à la pointe de sonde en injectant le fluide frigorigène liquide dans la pointe de sonde (26) ; et
maintenir la température de la pointe de sonde (26) à une température égale ou inférieure à la température de congélation en injectant le fluide frigorigène liquide dans la pointe de sonde (26) par le retrait de la pointe de sonde (26) du corps pour prélever le tissu et/ou les cellules cardiaques qui y adhèrent.

2. Appareil selon la revendication 1, dans lequel le premier dispositif de commande (24) est en outre configuré pour :
augmenter la température de la pointe de sonde au-dessus du point de congélation pour libérer le tissu ou les cellules cardiaques prélevés de la pointe de sonde une fois la pointe de sonde retirée du corps.

3. Appareil selon la revendication 1, dans lequel la pointe de sonde (26), ou une partie sur celle-ci, est détachable de la sonde (10) avec le tissu ou les cellules cardiaques prélevés une fois que la pointe de sonde (26) est retirée du corps.

4. Appareil selon la revendication 1, dans lequel la cryosonde comporte en outre :
une extrémité proximale comprenant un orifice d'entrée (18) et un orifice de sortie (19) ;
l'au moins un microtube conçu pour injecter le fluide frigorigène liquide reçu par le biais de l'orifice d'entrée (18) dans la pointe de sonde (26),
dans lequel la pointe de sonde (26) définit une chambre conçue pour recevoir le fluide frigorigène liquide en provenance du microtube et le fluide frigorigène liquide passe d'un
état liquide à un état gazeux lorsque le fluide frigorigène liquide quitte le microtube
et entre dans la chambre pour abaisser la température de la pointe de sonde (26), et dans lequel la sonde (10) définit une voie d'évacuation de gaz de la pointe de sonde vers
l'orifice de sortie.

5. Appareil selon la revendication 1, dans lequel le dispositif de commande (24) est en outre configuré pour commander la température de la pointe de sonde (26).

6. Appareil selon la revendication 5, dans lequel la pointe de sonde (26) comporte au moins un capteur (30) couplé au processeur et configuré pour détecter au moins une condition au niveau de la pointe de sonde (26).

7. Appareil selon la revendication 6, dans lequel l'au moins une condition au niveau de la pointe de sonde (26) est une température et/ou la présence d'un échantillon de tissu.

8. Appareil selon la revendication 6, dans lequel le dispositif de commande (24) et l'au moins un capteur (30) sont configurés pour détecter une température de la pointe de sonde (26) et/ou du fluide frigorigène entrant dans la pointe de sonde (26) et/ou sortant de celle-ci.

9. Appareil selon la revendication 6, dans lequel le dispositif de commande (24) et l'au moins un capteur (30) sont configurés pour détecter la présence et/ou l'absence du tissu ou des cellules cardiaques adhérant à la pointe de sonde (26).

10. Appareil selon la revendication 6, dans lequel le dispositif de commande (24) et l'au moins un capteur (30) sont configurés pour détecter la présence et/ou l'absence du tissu ou des cellules cardiaques adhérant à la pointe de sonde (26) à l'aide d'un capteur optique.

11. Appareil selon la revendication 6, comprenant en outre une boucle de rétroaction entre l'au moins un capteur (30) et le dispositif de commande (24) pour permettre au dispositif de commande (24) de surveiller et de réguler la température de la pointe de sonde (26).

12. Appareil selon la revendication 6, dans lequel le dispositif de commande (24) est configuré pour maintenir la température de la pointe de sonde (26) à la température de congélation ou en dessous de celle-ci après sa sortie du corps pendant au moins 10 secondes, au moins 30 secondes, ou au moins 1 minute.

13. Appareil selon la revendication 6, dans lequel le dispositif de commande (24) est configuré pour maintenir la température de la pointe de sonde (26) à la température de congélation ou en dessous de celle-ci après sa sortie du corps jusqu'à ce qu'une entrée d'utilisateur reçue par le biais d'une interface utilisateur ordonne au dispositif de commande (24) de permettre à la température de la pointe de sonde (26) de s'élever au-dessus de la température de congélation.

14. Appareil selon la revendication 6, dans lequel le dispositif de commande (24) comprend un capteur de température ambiante et le dispositif de commande (24) est en outre configuré pour régler la température de la pointe de sonde (26) à l'intérieur de la cavité cardiaque sur la base d'une température ambiante détectée à l'extérieur du corps.

15. Appareil selon la revendication 14, dans lequel le dispositif de commande (24) est en outre configuré pour régler la température de la pointe de sonde (26) en dessous de la température de congélation sur la base de la température ambiante détectée et d'une durée pendant laquelle la pointe de sonde (26) doit être à la température de congélation ou en dessous de celle-ci à l'extérieur du corps.
